# EUROPÄISCHE PATENTSCHRIFT

(11) **EP 1 343 902 B1**
(45) Veröffentlichungstag und Bekanntmachung des Hinweises auf die Patenterteilung: **10.04.2013**
(21) Anmeldenummer: 01985372.0
(22) Anmeldetag: 06.12.2001
(51) Int. Cl.: C12N 15/55

(54) **REKOMBINANTE SCHWEINELEBERESTERASEN, DEREN VERWENDUNG SOWIE EIN VERFAHREN ZU DEREN HERSTELLUNG**
RECOMBINANT PORCINE LIVER ESTERASES, THEIR USE AND A METHOD FOR THE PRODUCTION THEREOF
ESTERASES RECOMBINEES DE FOIE DE PORC, LEUR UTILISATION ET PROCEDE PERMETTANT DE LES PRODUIRE

(30) Priorität: 12.12.2000 DE 10061864
(43) Veröffentlichungstag der Anmeldung: 17.09.2003
(73) Patentinhaber: Enzymicals AG, 17489 Greifswald (DE)
(72) Erfinder: BORNSCHEUER, Uwe, 17489 Greifswald (DE); MUSIDLOWSKA, Anna, 17489 Greifswald (DE); SCHMIDT-DANNERT, Claudia, Shoreview, MN 55126 (US); LANGE, Stefan, 70499 Stuttgart (DE)
(74) Vertreter: Wachenfeld, Joachim
(86) Internationale Anmeldenummer: PCT/EP2001/014338
(87) Internationale Veröffentlichungsnummer: WO 2002/048322

(56) Entgegenhaltungen:
- WO-A-00/04160
- MATSUSHIMA M ET AL: "The nucleotide and deduced amino acid sequences of porcine liver proline- beta-naphthylamidase. Evidence for the identity with carboxylesterase." FEBS LETTERS. NETHERLANDS 18 NOV 1991, Bd. 293, Nr. 1-2, 18. November 1991 (1991-11-18), Seiten 37-41, XP002209303 ISSN: 0014-5793 in der Anmeldung erwähnt
- DAVID L ET AL: "Purification and molecular cloning of porcine intestinal glycerol-ester hydrolase--evidence for its identity with carboxylesterase." EUROPEAN JOURNAL OF BIOCHEMISTRY / FEBS. GERMANY 1 OCT 1998, Bd. 257, Nr. 1, 1. Oktober 1998 (1998-10-01), Seiten 142-148, XP002209304 ISSN: 0014-2956 in der Anmeldung erwähnt
- HEIM J ET AL: "Functional expression of a mammalian acetylcholinesterase in Pichia pastoris: comparison to acetylcholinesterase, expressed and reconstituted from Escherichia coli." BIOCHIMICA ET BIOPHYSICA ACTA. NETHERLANDS 13 MAR 1998, Bd. 1396, Nr. 3, 13. März 1998 (1998-03-13), Seiten 306-319, XP001059008 ISSN: 0006-3002
- GIVER L ET AL: "Directed evolution of a thermostable esterase." PROCEEDINGS OF THE NATIONAL ACADEMY OF SCIENCES OF THE UNITED STATES OF AMERICA. UNITED STATES 27 OCT 1998, Bd. 95, Nr. 22, 27. Oktober 1998 (1998-10-27), Seiten 12809-12813, XP002209305 ISSN: 0027-8424
- ROBBI M ET AL: "NUCLEOTIDE SEQUENCE OF CDNA CODING FOR RAT LIVER PI 6.1 ESTERASE (ES-10), A CARBOXYLESTERASE LOCATED IN THE LUMEN OF THE ENDOPLASMIC RETICULUM", BIOCHEMICAL JOURNAL, THE BIOCHEMICAL SOCIETY, LONDON, GB, vol. 269, no. 2, 15 July 1990 (1990-07-15) , pages 451-458, XP001053069, ISSN: 0264-6021
- OZOLS J: "Isolation, properties and the complete amino acid sequence of a second form of 60-kDa glycoprotein esterase orientation of the 60-kDa proteins in the microsomal membrane", JOURNAL OF BIOLOGICAL CHEMISTRY, AMERICAN SOCIETY FOR BIOCHEMISTRY AND MOLECULAR BIOLOGY, vol. 264, no. 21, 1 January 1989 (1989-01-01), pages 12533-12545, XP002188121, ISSN: 0021-9258
- KORZA G ET AL: "Complete covalent structure of 60-kDa esterase isolated from 2,3,7,8-tetrachlorodibenzo-p-dioxin-induce d rabbit liver microsomes", JOURNAL OF BIOLOGICAL CHEMISTRY, AMERICAN SOCIETY FOR BIOCHEMISTRY AND MOLECULAR BIOLOGY, vol. 263, no. 7, 5 March 1988 (1988-03-05) , pages 3486-3495, XP002188120, ISSN: 0021-9258

## Beschreibung

Die Erfindung betrifft die in den Ansprüchen beschriebenen Ausführungsformen.

Lipasen und Esterasen lassen sich als effiziente Biokatalysatoren zur Darstellung einer Vielzahl optisch aktiver Verbindungen einsetzen. Während jedoch eine ganze Reihe von Lipasen - insbesondere mikrobiellen Ursprungs - kommerziell erhältlich sind, gibt es nur sehr wenige Esterasen, die für den Einsatz in einer Racematspaltung bzw. Asymmetrisierung zur Verfügung stehen.

Obwohl gezeigt werden konnte, dass mit Esteraseextrakten aus Schweinelebergewebe teilweise Substrate mit genügender Stereoselektivität umgesetzt werden können, ist die Verwendung solcher Extrakte mit einer Reihe von Nachteilen verbunden. Neben Schwankungen des Esteraseanteils zwischen verschiedenen Chargen ist insbesondere die Anwesenheit weiterer Hydrolasen als problematisch bezüglich der Stereoselektivitäten anzusehen (Seebach, D. et. al., Chimia (1986), 40, 315-318). Zusätzlich besteht das Problem, dass die herkömmlichen Extrakte in Form mehrerer Isoenzyme vorliegen (Farb, D., et. al., Arch. Biochem. Biophys. (1980) 203, 214-226) die sich teilweise in ihrer Substratspezifität erheblich unterscheiden. Heymann, E. und Junge, W. (Eur. J. Biochem. (1979), 95, 509-518; Eur. J. Biochem. (1979), 95, 519-525) gelang eine aufwendige elektrophoretische Trennung, so dass Fraktionen isoliert werden konnten, die bevorzugt Butyrylcholin, Prolin-β-naphthylamid und Methylbutyrat spalten. Im Gegensatz dazu zeigen andere Untersuchungen (z. B. Lam, L.K.P., et. al., J. Am. Chem. Soc. (1988) 110, 4409-4411) lediglich Unterschiede in der Aktivität, nicht aber in der Spezifität einzelner Fraktionen.

Aus diesem Grunde besteht ein Bedarf an biotechnologisch hergestellten Schweineleberesterasen mit spezifischer Zusammensetzung.

Die Klonierung putativer Schweineleberesterase-Gene ist zwar bereits gelungen (Takahashi, T, et. al., J. Biol. Chem. (1989), 264, 11565-11571; FEBS Lett. (1991), 280, 297-300; FEBS Lett. (1991), 293, 37-41; Ldavid, L., et. al., Eur. J. Biochem. (1998) 257, 142-148), aber die funktionelle Expression eines aktiven Schweineleberesterase-Enzyms ist bisher, trotz der bestehenden Nachfrage nach solchen Enzymen, nicht gelungen.

Aufgabe der vorliegenden Erfindung war es somit, eine biotechnologisch einfach herstellbare, enzymatisch aktive Schweineleberesterase bereitzustellen.

Die Aufgabe wurde durch Schweineleberesterasen gelöst, deren monomere Untereinheiten am C-terminalen Ende gegenüber den natürlich vorkommenden Schweineleberesterase-Untereinheiten verkürzt ist. Darüber hinaus hat es sich als zusätzlich vorteilhaft erwiesen, das N-terminale Ende ebenfalls zu verkürzen.

Es konnte überraschend gezeigt werden, dass die biotechnologische Expression unter Sekretion dieser Untereinheiten der Schweineleberesterase in das Medium, zu enzymatisch aktiven rekombinanten Schweineleberesterasen führt, die weitgehend verunreinigungsfrei isoliert werden können, die hoch enantioselektiv arbeiten und darauf basierend reproduzierbare Ergebnisse bei der Substratumsetzung, insbesondere bezüglich der Ausbeute und Enantioselektivität, gewährleisten.

Somit sind ein Gegenstand der vorliegenden Erfindung rekombinante Untereinheiten der Schweineleberesterasen, denen am C-terminalen Ende 3 bis 10 Aminosäuren und besonders bevorzugt 3 oder 4 Aminosäuren, gegenüber den natürlich vorkommenden Schweineleberesterasen fehlen. Die erfindungsgemäßen Untereinheiten können am C-Terminus weitere funktionelle Peptiddomänen enthalten, so z. B. myc-Tags und/oder poly-His-Tags zur leichteren Isolierung über Affinitätschromatographie.

Weiterhin ist für die funktionsfähige biotechnologische Herstellung vorteilhaft, einen Bereich von 10 bis 50 Aminosäuren, bevorzugt 15 bis 25 Aminosäuren, am N-terminalen Ende gegenüber den natürlich vorkommenden Genprodukten zu entfernen.

Der N-Terminus kann ebenfalls funktionelle peptidische Domänen enthalten, dabei sind insbesondere Sekretionssignaldomänen von Interesse, wie die α-Faktor-Signalsequenz, die z. B. in der entsprechenden Primersequenz PLE-7F (**Seq. Id. No. 8**) enthalten ist, oder die ompA-Signalsequenz, die z. B. in der Primersequenz PLE-9F (**Seq. Id. No. 10**) enthalten ist.

Insbesondere Schweineleberesterasen-Untereinheiten, die ausgehend von einer putativen Schweineleberesterasen-Untereinheit gemäß Swiss-Prot Acc. No. Q29550 erhalten werden können, hierbei vor allem eine Schweineleberesterasen-Untereinheit mit der Aminosäuresequenz **Seq. Id. No. 1** deren funktionelle mutante Formen mit einer Sequenzhomologie von über 80%, insbesondere von über 90%, die auch biotechnisch erzeugt sein können, sind bevorzugt.

Unter einer funktionellen mutanten Form ist im Sinne dieser Erfindung eine Schweineleberesterase-Untereinheit zu verstehen, die sich zu enzymatisch aktiven rekombinanten Schweineleberesterasen zusammenlagern kann. Darunter fallen auch die Untereinheiten, die von den natürlich vorkommenden Monomeren der Schweineleberesterasen des Typs α, β, γ ableitbar sind. Bevorzugt fallen künstlich erzeugte mutante Formen darunter, die in der Umgebung des aktiven Zentrums des resultierenden Enzyms verändert wurden. Dies betrifft z. B. Mutationen in der Umgebung der Positionen Asp80, Ser 186 und His431 der monomeren Untereinheit gemäß **Seq. Id. No. 1.** Dadurch kann die enzymatische Aktivität und Selektivität der rekombinanten Schweineleberesterase bezüglich eines bestimmten Substrates gesteigert werden.
Unter den Begriff der Schweineleberesterase-Untereinheit fallen ferner jene Derivate des ursprünglichen Translationsproduktes die durch posttranslationale Modifikation aus ihnen hervorgehen.

Die einzelnen monomeren Untereinheiten der rekombinanten Schweineleberesterase können in Lösung zu einem besonders funktionsfähigen Enzym multimerisieren. Dabei können auch Untereinheiten eines unterschiedlichen Typs miteinander in Verbindung treten. Darüber hinaus zeigen auch die Monomere selbst eine enzymatische Aktivität, wenn auch in aller Regel schwächer als die Multimere.

Die so gewonnenen Schweineleberesterasen zeichnen sich durch eine hohe Reinheit aus. Insbesondere können so die üblichen, in Schweineleberesterase-Extrakten vorkommenden Verunreinigungen durch Isoenzyme und andere Hydrolasen vermieden werden. Die rekombinanten Schweineleberesterasen zeigen weiterhin eine erhöhte, teilweise sogar eine stark erhöhte Enantioselektivität, was deren Einsatz in organisch-enzymatischen Synthesen interessant macht. Von besonderem Interesse ist auch, daß die rekombinanten Schweineleberesterasen mitunter eine gegenüber kommerziell erhältlichen Schweineleberesterasen entgegengesetzte Stereopräferenz zeigen. Die rekombinanten Schweineleberesterasen sind darüber hinaus in einer gleichbleibender Qualität herstellbar.

Ein weiterer Gegenstand der vorliegenden Erfindung sind Nukleinsäuren, codierend für die erfindungsgemäßen rekombinanten Schweineleberesterase-Untereinheiten oder DNA-Fragmente, die zu solchen Nukleinsäuresequenzen komplementär sind, welche mit diesen codierenden Nukleinsäuren unter stringenten Bedingungen hybridisierenden. Dazu können gängige Hybridisierungsbedingungen (z. B. 60°C, 0,1xSSC, 0.1 % SDS) genutzt werden.

In einer weiteren bevorzugten Ausführungsform wird zur gezielten Termination der Translation am 3'Ende des codierenden Bereichs ein Stop-Codon eingeführt.

Die codierenden DNA-Sequenzen können in herkömmliche Vektoren kloniert und nach Transfektion von Wirtszellen mit solchen Vektoren in Zellkultur exprimiert werden. Ein geeigneter Vektor ist z. B. der pUC19-Vektor oder der pCYTEX-Vektor für die Transformation von *E*. *coli* oder der pPICZα-Vektor für die Transformation der Hefe *Pichia pastoris.* Innerhalb der Species *Aspergillus sp., Schwanniomyces sp., Kluyveromyces sp., Yarrowia sp., Arxula sp., Saccharomyces sp., Hansenula sp. oder Pichia sp.* sind weitere interessante einzellige Organismen, die sich als Wirt für die biotechnologische Expression rekombinanter Enzyme als geeignet erwiesen haben. Als bevorzugte Wirtsorganismen sind, neben *P. pastoris Saccharomyces cerevisiae, Aspergillus orycae, Schwanniomyces occidentalis, Kluyveromyces lactis, Yarrowia lipolytica, Arxula adeninivrans, Pichia methanolica, Pichia guilliermondii.* oder *Hansenula polymorpha* zu nennen.

Die codierenden DNA-Fragmente müssen in den Vektoren im offenen Leseraster zu einem Promotor liegen. Als Promotoren sind vor allem starke Promotoren, wie z. B. der lac-, lambda-, T7- ,T4-, der Rhamnose induzierbare-Promotor oder der Alkoholoxidase (AOXI)-Promotor, bevorzugt. Die Vektoren können weitere funktionelle Bereiche enthalten. Neben den Selektionsmarkern und Replikationsstartpunkten sind vor allem genregulatorische Elemente, wie z. B. Operatoren, Repressoren oder Transkriptionsfaktoren von Interesse. Insbesondere eine Konstruktion von Vektoren, die eine reversible, induzierbare oder reprimierbare Expression der rekombinanten Schweineleberesterase-Untereinheiten oder des funktionsfähigen Enzyms selbst zulässt, können eingesetzt werden.
Bevorzugte Wirtszellen zur Transfektion mit Vektoren, enthaltend die erfindungsgemäßen codierenden DNA-Fragmente zur Expression von Schweineleberesterase-Untereinheiten, sind einzellige pro- oder eukaryotische Organismen, wie z. B. *Aspergillus sp., S. cerevisiae, Hansenula sp., E. coli* oder *P*. *pastoris.*

Somit ist ein weiterer Gegenstand der vorliegenden Erfindung die Verwendung von DNA-Fragmenten, die für eine erfindungsgemäße Schweineleberesterase-Untereinheit und gegebenenfalls für weitere daran fusionierte N- und/oder C-terminale Domänen codieren, zur Klonierung in Vektoren, die Verwendung dieser Vektoren zur Transformation von Zellen sowie die Verwendung solcher transformierten Zellen oder Zellkulturen zur Expression der rekombinanten Schweineleberesterase-Untereinheiten. Die exprimierten Untereinheiten können z. B. in monomerer Form isoliert werden, aber auch die Multimerisierung der Untereinheiten zu enzymatisch aktiven rekombinanten Schweineleberesterasen im Medium und anschließender Isolierung des funktionsfähigen Enzyms ist möglich.

Bevorzugt ist die Kultivierung der Wirtszellen und die sekretorische Expression der Schweineleberesterase-Untereinheiten in Flüssigkulturbehältern in einem steadystate- Verfahren.

Die Aktivität der exprimierten Esterase kann photometrisch durch die Umsetzung ausgewählter Substrate überprüft werden. Als geeignet hat sich dabei die Esterase-katalysierte Chromophorenspaltung von Acetaten, wie z. B. p-Nitrophenylacetat, erwiesen.

Neben der biotechnologischen Expression von enzymatisch aktiven rekombinanten Schweineleberesterasen können die DNA-Fragmente, die Expressionsvektoren oder Expressionssyteme zur Mutagenese verwendet werden. Die dann exprimierbaren mutanten Schweineleberesterasen und deren monomere Untereinheiten können auf ihre enzymatische Aktivität getestet und selektiert werden. Durch mehrfache Wiederholung des Mutagenese-, Expressions- und Selektionszyklus lassen sich maßgeschneiderte enzymatische Katalysatoren für die organische Synthese erzeugen. Der Selektionsschritt kann dabei durch Zugabe der exprimierten rekombinanten Enzyme zu einem, ein potentielles Substrat enthaltenden Reaktionsansatz erfolgen, wobei der Umsatz z. B. photometrisch bestimmt werden kann.

Als Substrat für die katalytische Umsetzung mittels rekombinanter Schweineleberesterase kommen vor allem aromatisch-aliphatische und aliphatischaliphatische Ester in Frage, hierbei vor allem Carbonsäureester chiraler oder prochiraler Alkohole, wobei die Carbonsäurekomponente bevorzugt 2 bis 6, besonders bevorzugt 2 bis 4 C-Atome umfaßt und auch verzweigt sein kann.
Die enzymatisch-katalytische Racematspaltung der Carbonsäureester, insbesondere der Acetate, verläuft dabei mit hoher bis ausgezeichneter Enantioselektivität.
Das Optimum der Enzymaktivität von rekombinanten Schweineleberesterasen, die monomere Untereinheiten gemäß **Seq. Id. No. 1** enthalten, liegt bei einem pH zwischen 5 und 10, bevorzugt zwischen 7 und 9, und bei einer Temperatur zwischen 20°C und 90°C, bevorzugt zwischen 30°C und 80°C, besonders bevorzugt zwischen 40 oder 50°C und 60 oder 70°C.

Weiterhin können die erfindungsgemäßen rekombinanten Enzyme auch bei der Racematspaltung von Carbonsäuren oder bei der Umsetzung von prostereogenen Verbindungen, insbesondere von Diolen oder Dicarbonsäuren eingesetzt werden.

Im folgenden werden zur Verdeutlichung der Erfindung einige Ausführungsbeispiele gegeben, die allerdings nicht als einschränkend aufzufassen sind.

Allgemeines, Verwendete Mikroorganismen, Medien, Vektoren und Oligonukleotide

Der *E*. *coli* Stamm DH5α (F⁻ *end*A1 hsdR17(rk⁻, mk⁺)*sup*E44 *rhi-*I λ *gyr*A96 *rel*/Al Δ(*arg*F-*laczya*)U169) wird zur Erhaltung und Vermehrung der im folgenden verwendeten Plasmide genutzt. Der *E*. *coli* Stamm DH5α wird in LB_{lowsalt} (10gl⁻¹ Hefeextrakt, 1010gl⁻¹ Peptone und 5gl⁻¹ NaCl) kultiviert. Die Kulturlösung wird mit 50mgl⁻¹ Nalidixinsäure und gegebenenfalls mit 100mgl⁻¹ Ampicillin oder 25mgl⁻¹ Zeocin (Invitrogen, Carlsbad, CA, USA) versetzt.

Für die Expressionsversuche wurde die Hefe *Pichia pastoris* X33 (Invitrogen) genutzt. Zur Kultivierung der *Pichia*-Zellen wurden folgende Medien genutzt:
- YPD-Medium (1% Hefeextrakt, 2% Peptone und 2%Glucose);
- YPDS-Medium (YPD-Medium in 1 M Sorbitol);
- BMGY-Medium(1% Hefeextrakt, 2% Peptone, 100mM Kaliumphosphat pH 6, 1,34% Hefe-Stickstoffbase mit Ammoniumsulfat ohne Aminosäuren und 1 % Glycerol);
- BMMY-Medium (BMGY-Medium, wobei 0,5% filtersterilisiertes Methanol anstatt 1% Glycerol verwendet wird)

Die Medien werden mit 100mgl⁻¹ Zeocin versetzt. Für plattierbare Medien wird 1,5% Agar hinzugesetzt.

Der *E*. *coli - P. pastoris* Vektor pPICZαA (Invitrogen) wurde verwendet, um eine DNA-Sequenz codierend für eine Schweineleber-Carboxylesterase zu klonieren, die unter der Kontrolle des Alkoholoxidase-Promotors AOXI steht.
Die für die PCR verwendeten Oligonukleotidprimer sind in Tabelle 1 zusammengestellt.

**Tabelle 1**

| Oligonukleotid | Nukleotidsequenz | Bemerkung |
|---|---|---|
| PLE-1F | | *Eco*RV, *Sma*I *Nde*I |
| PLE-2R | | *Sma*I, *Eco*RI |
| PLE-3F | | - |
| PLE-4R | | - |
| | | |
| LE-5F | | - |
| PLE-6R | | - |
| PLE-7F | | EcoRI |
| PLE-8R | | XbaI |
| PLE-9F | | EcoRI |
| PLE-10R | | XbaI |
| PLE-11R | | XbaI |

### Allgemeines: DNA-Rekombination und Transformation

Sofern nichts anderes erwähnt, werden Standardverfahren nach Sambrook, J., Fritsch, E., Maniatis, T. (1989) Molecular Cloning: A Laboratory Manual, 2nd Edn., Cold Spring Habour, NY verwendet.
Zur Plasmid und DNA Extraktion wird ein QIAprep Spin Miniprep Kit, ein Plasmid Midi Kit oder ein QIAquick Gel Extraction Kit (Qiagen, Hilden, Germany) verwendet.
Die eingesetzten Restriktionsenzyme werden gemäß den jeweiligen Herstellerangaben verwendet. Die DNA-Sequenzierung erfolgt anhand beider Stränge mit Hilfe des Taq Ready Reaction Dye Deoxy^{™} Terminator Cycle Sequencing Kit (Applied Biosystems, Weiterstadt, Germany). Die Sequenzierungsprodukte werden mit einem 373 DNA-Sequencer der gleichen Firma analysiert.
Für die Preparation und Transformation von competenten *E*. *coli* -Zellen wird ein Standardprotokoll gemäß Chung, C.T., Niemela, S.L. & Miller, R.H. (1989) Proc. Natl. Acad. Sci. USA. 86, 2172-2175, benutzt. Die Transformation von *P. pastoris* erfolgt mit den jeweiligen Plasmiden durch Elektroporation.

### Beispiel 1: Gewebepräperation, mRNA Isolierung, und Klonierung der cDNA

0,8g frischen Schweinelebergewebes werden homogenisiert und die freigesetzte polyA-mRNA unter Verwendung des Fast Track 2.0 Kit (Invitrogen) gemäß den vom Hersteller gemachten Angaben isoliert. Die cDNA-Synthese auf Basis des mRNA-Extraktes wurde durch RT-PCR mit Hilfe des cDNA Cycle^{™} Kits (Invitrogen) unter Einsatz von oligo-dT-Primern gemäß dem Kit-Protokoll durchgeführt. Die RT-PCR Produkte sind dann als Template für die Amplifikation der Schweineleberesterase (pig liver esterase PLE) herangezogen worden. Als genspezifische Primer fanden dabei die Oligonukleotide PLE-1 F (**Seq. Id. No. 2**) und PLE-2R (**Seq. Id. No. 3**) (Tabelle 1) Verwendung, wodurch gleichzeitig zur Klonierung des PCR-Produktes benötigte Restriktionsschnittstellen eingeführt wurden. Beide Primer wurden anhand der mRNA-Sequenz der Schweineleber Prolin--Naphtylamidase (Matsushima, M., et. al. (1991) FEBS Lett. 293, 37-41) hergestellt von der vermutet wurde, dass sie eine der PLE komplementäre Sequenz besitzt. Die PCR wurde in einem Thermocycler (Robocycle Gradient 40, Stratagene, La Jolla, CA, USA) durchgeführt. Nach einem ersten fünfminütigen Denaturierungsschritt bei 95°C wurden 28 Zyklen gemäß dem folgenden Temperaturprogramm durchgeführt: 1 min 95°C, 2min 55°C und 3min 72°C.
Das aufgereinigte PCR-Produkt wird mit seinen glatten Enden in die *Sma*I-Restriktionsschnittstelle des pUC19-Vektors kloniert. Der resultierende pUC19-PLE-R-Vektor wurde zur Transformation von *E*. *coli* DH5α-Stämmen benutzt, das Plasmid wurde durch Kultivierung der transformierten Stämme vermehrt. Das Plasmid wurde aus den rekombinanten *E*. *coli* Stämmen isoliert, über Größenbestimmung bzw. Restriktions-Mapping identifiziert und als Template für die PCR-Amplifikation der PLE-Sequenz herangezogen. Die amplifizierte PLE-Sequenz wurde anschließend sequenziert.

### Beispiel 2: Konstruktion eines Expressionsvektors für E. coli und für P. pastoris:

Für die intrazellulare Expression der klonierten PLE in *E*. *coli* wurde der pUC19-PLE-R Vektor mit *Nde*I und *Eco*RI geschnitten, dadurch wird ein 1705bp langes Fragment codierend für die komplette natürlich vorkommende PLE-Sequenz erhalten. Das erhaltene Restriktionsfragment wird in die *Nde*I/*Eco*RI-Restriktionsschnittstelle von pT1-BTL2 (Belev, T.N. et. al. (1991) Plasmid, 26, 147-150) insertiert, wobei sich ein neuer Vektor pCYTEX-PLE (6652bp) bildet.
Für die periplasmatische Expression von PLE-Sequenzen ohne die N-terminale Leadersequenz (mPLE) wurde die entsprechende DNA-Sequenz mit den Primem PLE-3F (**Seq. Id. No. 4**) und PLE-4R (**Seq. Id. No. 5**) (Tabelle 1) amplifiziert und mit ihrem glatten Ende in die pT1-ompA Sequenz des pT1-ompA-BTL2-Vektors (Rua, M.L., et. al. Appl. Microbiol. Biotechnol. 49, 405-410) ligiert, der mit Hilfe der Primer PLE-5F (**Seq. Id. No. 6**) und PLE-6R (**Seq. Id. No. 7**) (Tabelle 1) ebenfalls amplifiziert werden kann. Der resultierende Vektor (der das BTL2-Gen nicht mehr enthält) wird als pCYTEX-ompA-mPLE (6682bp) bezeichnet, der das mPLE-Gen im offenen Leseraster zur ompA-Leadersequenz unter der Kontrolle eines durch Hitze induzierbaren λ-Promotors enthält.
Für die Expression des mPLE-Gens in *P. pastoris,* wurde die mPLE DNA-Sequenz im offenen Leseraster hinter einen α-Faktor-Signalsequenz des *E*. *coli - P. pastoris* Vektors pPICZαA (von Invitrogen) kloniert. Das mPLE-Gen wurde dann mit den Primem PLE-7F (**Seq. Id. No. 8**) und PLE-8R (**Seq. Id. No. 9**) (Tabelle 1) amplifiziert, das Amplifikationsprodukt wird mit den Restriktionsenzymen Ndel und Xbal geschnitten und in die entsprechende Restriktionsschnittstelle des pPICZαA-Vektors ligiert. Der resultierende Vektor wird mit pPICZα-mPLE bezeichnet.

Weiterhin wurden Expressionsvektoren konstruiert, deren Expressionsprodukt eine Deletion des HAEL-Tetrapeptids am C-terminalen Ende aufweist. Dazu wurde das mPLE-Gen mittels der Primer PLE-9F (**Seq. Id. No. 10**) und PLE-10R (**Seq. Id. No. 11**) oder PLE-11R (**Seq. Id. No. 12**) amplifiziert. Beide PCR-Produkte wurden in die *Eco*RI/*Xba*I-Restriktionsschnittstelle von pPICZαA ligiert. Die Fragmente, erzeugt mit Hilfe des Primers PLE-10R, führen zu einem Fusionsprotein enthaltend myc- und His-Tag-Domänen, die auf dem ursprünglichen pPICZαA-Vektor bereits codiert sind. Das PCR-Produkt erhältlich durch Verwendung des Primers PLE-11 enthält keine C-terminalen Tags, da durch den Primer ein Stop-Codon eingeführt wird, das die Fusion mit den Tag-Domänen des pPICZαA-Vektors verhindert.

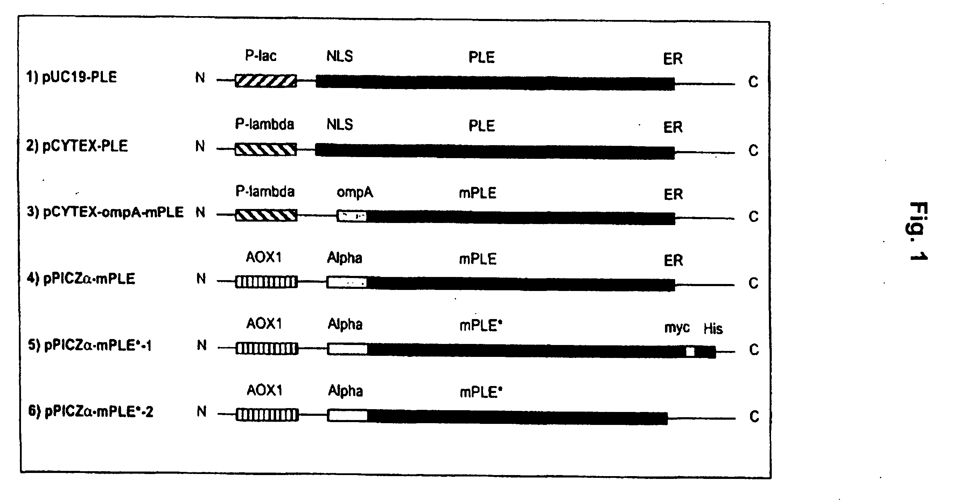

Alle resultierenden Vektoren pPICZα-mPLE (5183bp), pPICZα-mPLE*-1 (5170bp) und pPICZα-mPLE*-2 (5171 bp) werden an der *Pme*I Restriktionsschnittstelle linearisiert und in *P. pastoris* mittels Elektroporation (Invitrogen) eingeschleust.
Die hergestellten Vektoren sind schematisch in **Fig. 1** wiedergegeben.

### Beispiel 3: Expression von rekombinanter PLE in E. coli DH5α

Rekombinante *E*. *coli* DH5α, die mit dem pCYTEX-PLE oder dem PCYTEX-ompA-mPLE Vektor transformiert sind werden bei 37°C, 200rpm in LB-Medium kultiviert bis eine Zelldichte von OD₅₇₈ von 0,8 bis 1,0 erreicht ist. Die Expression des rekombinanten Proteins wurde durch Temperaturerhöhung auf 42°C induziert. Jede Stunde wurde eine Probe entnommen, 3 bis 4 Stunden nach der Induzierung wurden die Zellen geerntet und bei -20°C gelagert bzw. direkt mittels SDS-PAGE oder einem Aktivitäts-Assay untersucht.

### Beispiel 4: Kultivierung und sekretorische Expression von rekombinanter PLE in P. pastoris

Recombinante Klone, selektiert auf Zeocin-Nährboden wurden gepickt und auf YPDS-Medium bei 30°C, 200rpm kultiviert bis eine Zelldichte von OD₆₀₀ von ca. 15 erreicht ist. 25ml eines BMGY-Mediums wurden mit 200µl dieser Kultur geimpft und über Nacht bei 30°C kultiviert. Danach wurden die Hefezellen durch fünfminütige Zentrifugation bei 3000g und 4°C isoliert und auf BMMY-Induktions-Medium transferiert, wobei die Kultur bis zu einer Zelldichte von OD₆₀₀ von 1,0 gezüchtet wird. Die Induktion wird durch tägliche Addition von 0,5% (v/v) Methanol vorgenommen. Nach 96-stündiger Induktion wurden die Zellen durch Zentrifugation geerntet. Die Überstände enthalten das rekombinante Enzym, das durch 15-minütiges Zentrifugieren bei 4000g und 4°C unter Zusatz von 20ml Centricons (NMWL 30000, Ultracel-PL membrane, Millipore) konzentriert wird. Die Bestimmung der Aktivität während der Wachstumsphase der Kultur und des aufkonzentrierten enzymhaltigen Mediums wurden mit einem pNPA-Assay bestimmt, die Proteine wurden mit Hilfe der Gelelektrophorese identifiziert (die nähere Beschreibung erfolgt im Anschluß). Als Referenz für die densitometrische Bestimmung der Proteinkonzentration wurden Serumalbumin in bekannten Konzentrationen verwendet.

### Beispiel 5: SDS-Polyacrylamidgel Elektrophorese

20µl kommerziell erhältlicher Schweineleber-Carboxylesterase (100U, nach pNPA-Assay) gelöst in 2ml oder 20µl der 10-fach konzentrierten Überstandes der *P*. *pastoris* Kultur wurden mit 10µl eines 2xSDS-Probenpuffers versetzt. Nach Erhitzen der Lösung 95°C für 5min, werden die Proteine auf einem 12,5%igen Polyacrylamidgel, 4%igen Stacking-Gel getrennt. Die Proben wurden zum Proteinnachweis mit Coomassie Brilliant Blue R250 angefärbt. Zur Esterase-Aktivitätsbestimmung wurden die Proteine 12 Stunden in einer Triton X-100 Lösung (0,5% in 0,1 M Tris/HCl pH7,5) renaturiert. Danach wurde das Gel mit einer 1:1-Mischung aus Lösung A (20mg α-Naphthylacetat gelöst in 5ml Aceton und darauffolgender Addition 50ml 0,1M Tris/HCl pH7,5, 50mg Fast Red TR Salz gelöst in 50ml 0,1 M Tris/HCl, pH7,5) und Lösung B (50mg Fast Red TR Salz gelöst in 50ml 0,1 M Tris/HCl, pH7,5) versetzt. In Gegenwart von hydrolytischer Lipase- oder Esteraseaktivität wird eine rote α-Naphthylform des Fast Red gebildet (Krebsfänger, N., et. al., (1998) Enzyme Microb. Technol. 22, 641-646).

### Beispiel 6: N-terminale Protein-Sequenzierung

Die kommerziell erhältliche PLE-Fraktion wird spariert und von einem SDS-PAGE-Gel auf eine PVDF-Membran geblottet (Matsudaira, P. (1987) J. Biol. Chem. 262, 10035-10038). Die Sequenzierung wird mit einem Gasphasen-Sequenzer durchgeführt.

### Beispiel 7: Bestimmung der Esteraseaktivität

Die Esteraseaktivität wird photometrisch in einem Natriumphosphat-Puffer (50mM, pH7,5) bestimmt. p-Nitrophenylacetat (10mM gelöst in DMSO) wird als Substrat verwendet. Die freigesetzte Menge an p-Nitrophenol wird bei 410nm (ε = 12.36x10³M⁻¹cm⁻¹) bei Raumtemperatur bestimmt. Die Enzymaktivität wurde zusätzlich bei unterschiedlichen pH-Werten bestimmt. Als Einheit U wird eine Esteraseaktivität definiert, bei der 1µmol p-Nitrophenol pro Minute unter Assay-Bedingungen umgesetzt wird. Die Substratspezifität von PLE wurde unter Verwendung eines pH-stat Assays ermittelt. Dazu wurden zu 30ml einer Emulsion enthaltend 5% (v/v) eines Esters (Methylbutyrat, Ethylcaprylat, Ethylacetat, Triolein, Tricaprylin) und 2% (w/v) Gum Arabic. Dazu wird, bei einer Temperatur von 37°C, eine definierte Menge an Esterase gegeben. Die freigesetzte Säure wird automatisch mit Hilfe eines pH-stat (Schott, Mainz, Germany) mit 0,01 N NaOH titriert, um einen konstanten pH-Wert von 7,5 aufrechtzuerhalten. Ein U entspricht einem Säureverbrauch 1µmol pro Minute unter Assay-Bedingungen. Die Aktivitätsbestimmung bei unterschiedlichen Temperaturen wurde mit Ethylcaprylat als Substrat bei pH7,5 durchgeführt. Die höchste ermittelte Aktivität wurde als 100%-Wert angenommen.

### Beispiel 8: Bestimmung der Prolin-ß-naphthylamidase-Aktivität

Die Prolin-ß-naphthylamidase-Aktivität wird photometrisch unter Verwendung von Prolin-ß-naphthylamid (0,2mM in DMSO) als Substrat bestimmt. Die Reaktion wird in einer Mischung enthaltend 0,1 M Tris/HCl Puffer pH8,0, 50µl Substrat durchgeführt und mit rekombinanten oder kommerziell erhältlichen PLE-Präparationen werden bei 37°C für 30min mit 0,4U (gemäß pNPA-Assay) versetzt. Die Reaktion wird mit durch Addition von 1,5ml Kupplungsreagenz FastGarnet (Sigma) abgebrochen (Barret, A.J., (1977) Proteinases in Mammalian Cells and Tissues Barret, A.J., ed pp. 181-208, Elsevier, Amsterdam). Die freigesetzte Menge an ß-Naphthylamin wird bei 520nm (ε = 24.03x10³M⁻¹cm⁻¹) bestimmt. Eine U Amidaseaktivität wird definiert als die Enzymmenge, die 11µmol β-Naphthylamin pro Minute unter Assay-Bedingungen freisetzt.

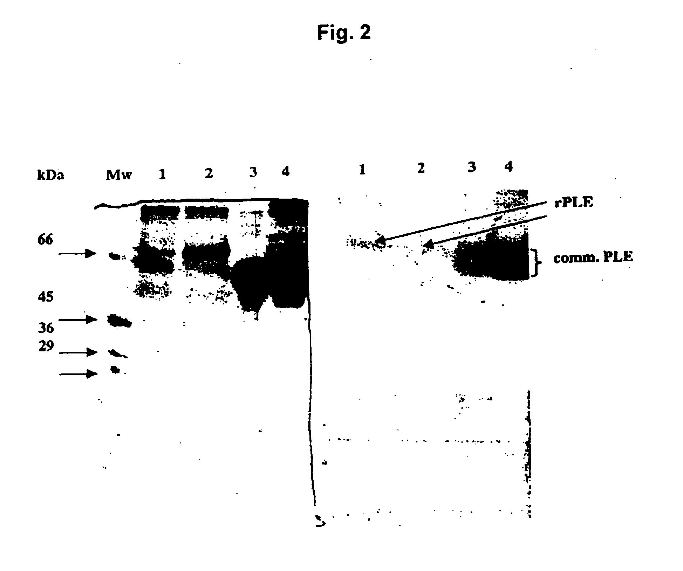

Die PLE-Expressionsprodukte der Vektoren pCYTEX-PLE enthaltend PLE mit der nativen Leadersequenz und pCYTEX-PLE-ompA-mPLE enthaltend die mPLE-Nukleinsäuresequenz fusioniert mit der ompA-Leadersequenz, die eine Sekretion des Esteraseenzyms in den periplasmatischen Raums gewährleisten sollte (**Fig.1**, 2) und 3) (Beer, H.D., et. al., Biochim. Biophys. Acta. 1399, 173-180) zeigen schematisch die Vektorkonstrukte). Keine der transfizierten Kulturen war allerdings in der Lage enzymatisch aktive native oder rekombinante Schweineleberesterase zu exprimieren, sowohl die Untersuchung mit der SDS-PAGE und dem Esterase-Aktivitäts-Assay waren negativ.

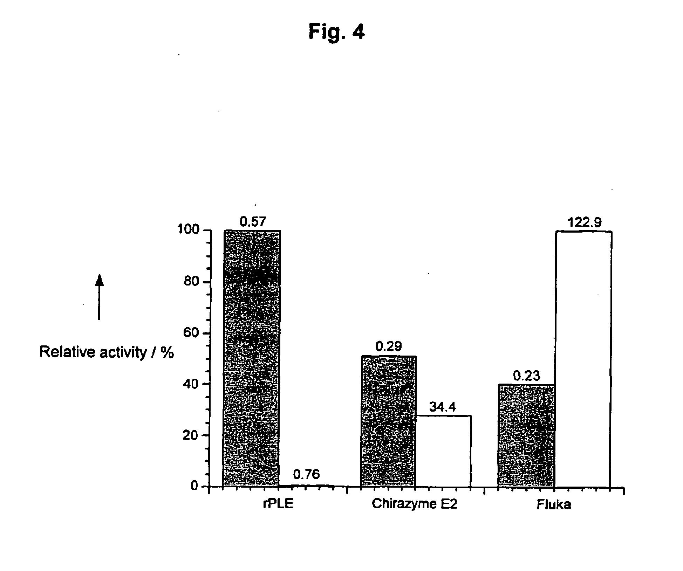

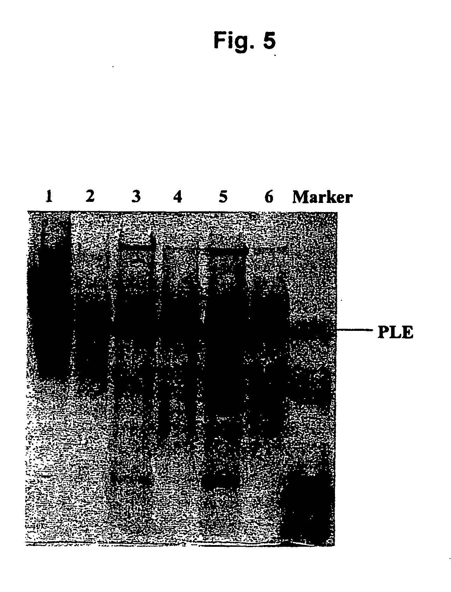

Zur Expression rekombinanter Schweineleberesterasen in *P. pastoris* werden Vektoren gemäß der schematischen Darstellung in **Fig.1**, 4) bis 6) herangezogen. Der Vektor dargestellt in **Fig.1**, 5) (pPICZα-mPLE*-1) enthält neben dem N-terminalen Sekretionssignal Alpha (α-Faktor), die C-terminalen Tags myc und His(6x), die beide vom Vektor pPICZαA stammen. Das Konstrukt 6) (pPICZα-mPLE*-2) dargestellt in **Fig.1** enthält keine C-terminalen Tags, da hier im Leseraster ein Stop-Codon vor den entsprechenden Vektorbereichen eingeführt wurde. Bei den Vektorkonstrukten fehlt am C-Terminus der in der nativen Schweineleberesterase vorkommende C-Terminus HAEL. Der dritte hergestellte *P. pastoris* -Vektor (pPICZα-mPLE, **Fig.1**, 4)) besitzt weiterhin am C-Terminus die natürliche Tetrapeptidsequenz HAEL (ER). Die Vektoren wurden linearisiert und mit ihnen *P*. *pastoris* transfiziert. Die Überstände von 30 Klonen wurde jeweils auf die aktive Expression des klonierten Enzyms mit Hilfe des pNPA-Assays untersucht. Im Falle des Vektors **Fig.1**, 4) konnte keine Esteraseaktivität festgestellt werden. Im Gegensatz dazu konnte gezeigt werden, dass durch die Verwendung von Vektoren gemäß der **Fig.1**, 5) oder 6), denen der codierende Bereich für den nativen C-Terminus fehlt, aktiv exprimiert und sezemiert werden können. Je ein Klon mit und ohne C-terminale Tags wurde gepickt und weiter charakterisiert.

Die Aktivität, der für weitere Untersuchungen benutzten Überstände wurde, nach 96-stündiger Kultivierung der gepickten Klone, mit Hilfe des pNPA-Assays auf 0,5 U/ml bestimmt. Nach der Konzentration des Enzyms durch Zentrifugation ergab sich eine Aktivität von 10U/ml, was einer spezifischen Proteinaktivität von ca. 500U/mg entspricht. Der Quotient Vₘₐₓ/Kₘ liegt bei der erfindungsgemäßen rekombinanten Schweineleberesterase bei 139 min⁻¹mg⁻¹ für pNPA als Substrat (Werte im Vergleich zu einem käuflich erhältlichen Schweineleberesterase-Extrakt sind in der folgenden Tabelle 2 aufgelistet).

**Tabelle 2:**

| Esterase | Vₘₐₓ (U/mg⁻¹) | Kₘ (mM) | Vₘₐₓ/Kₘ (min⁻¹mg⁻¹) |
|---|---|---|---|
| rekombinante | | | |
| PLE | 0,74x10³ | 5,32 | 139 |
| PLE käuflicher | | | |
| Extrakt (Fluka) | 1,58x10³ | 1,82 | 868 |

Die erfindungsgemäße rekombinante PLE spaltet Ethylcaprylat und Tributyrin mit einer Aktivität von etwa 50U/mg und Ethylacetat mit einer Aktivität von etwa 20U/mg. Triolein wird nicht gespalten, wie es für eine reine Esterase erwartet wird. Die käuflichen Extrakte (Fluka oder Roche (Chirazyme E-2)) hydrolysieren hingegen Triolein, was auf das vorliegen von Verunreinigungen durch Lipasen oder durch PLE-Isoenzyme hindeutet.

**Fig. 2** zeigt eine SDS-PAGE Analyse (links) und einen Aktivitätsnachweis (rechts). Das Molekulargewicht der erfindungsgemäßen rekombinanten Schweineleberesterase-Untereinheit (**Seq. Id. No. 1** mit C-terminalen Tags, Bahnen 1 und 2 in **Fig. 2**, Mw Molekulargewicht-Standard) liegt bei ca. 61 bis 62 kDa, bestimmt mit einer SDS-PAGE-Analyse. Die Aktivitätsanalyse mit Fast Red zeigt für die erfindungsgemäße rekombinante Schweineleberesterase (0,4U) eine scharfe Bande, wohingegen die käuflichen Extrakte verschmieren, was auf das vorliegen unterschiedlicher Isoenzyme bzw. anderer Hydrolasen zurückgeführt werden kann (4U PLE-Fluka, Bahnen 3, 4U PLE-Chirazyme-E2, Bahnen 4, **Fig. 2**).

Die Messung der enzymatischen Aktivität bei unterschiedlichen Temperaturen und pH-Werten bezüglich der Hydrolyse von Ethylcaprylat zeigt ein Optimum bei 60°C (pH7,5), wobei die erfindungsgemäße Esterase bei 70°C vollständig inaktiviert wird, und ein Optimum bei ca. pH8 (37°C) aufweist.

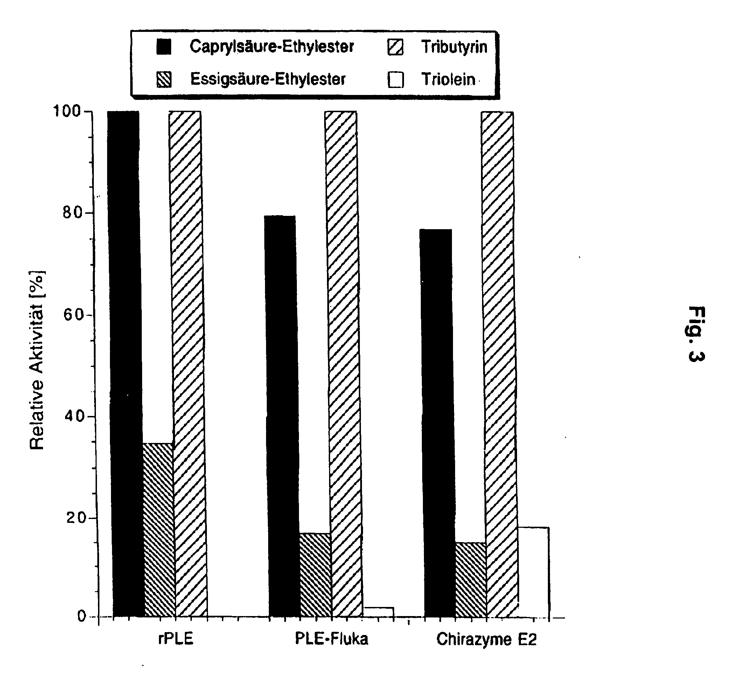

**Fig. 3** zeigt die relative Aktivität der erfindungsgemäßen rekombinanten PLE (rPLE) und der käuflichen PLE-Extrakte (PLE-Fluka, Chirazyme E2) bezüglich der Substrate Caprylsäure-Ethylester, Essigsäure-Ethylester, Tributyrin und Triolin.

**Fig. 4** zeigt die relativen Aktivitäten der Enzyme für die Substrate Prolin-β-naphthylamid (PNA, schwarzer Balken) und Methyl-Butyrat (weißer Balken).

### Beispiel 9: Weitere Charakterisieung der erhaltenen rekombinanten SchweineleberEsterase

Native-Polyacrylamid-Gelelektrophorese 10µl kommerziell erhältlicher PLE (0.1U) und 5-1µl der konzentrierten Überstände der *P. pastoris* Kulturen wurden mit 10 µl eines Probenpuffers gemischt. Die Proben wurden auf einem 7.5 %igen Polyacrylamidgel, 4%igen Stacking-Gel getrennt. Die Gele wurden aktivitäts- und danach Coomassie Brilliant Blue gefärbt.

**Fig. 5** zeigt die Ergebnisse der nativen PAGE von recombinanter und kommeziellen PLEs. Mw: Molekulargewichtsstandards: (272 kDa, Jack Bean Urease (trimer); 132 kDa (dimer) und 66 kDa (monomer), Bovine Serum Albumin; 45 kDa, Chicken Egg Albumin); Bahn 1: 0.1 U Fluka PLE; Bahn 2: 0.07 U Chirazyme E-2; Bahnen 3-6: 0.1, 0.045, 0.09 und 0.045U rPLE (Proben aus verschiedenen Kultivierungen), Units basieren auf einem pNPA-Test.

Isoelektrische Focussierung 1-5µl der konzentrierten Überstände der *P. pastoris* Kulturen (0.02 - 0.1U) wurden mit 10 µl eines Probenpuffers gemischt. Die Proben wurden auf einem 5 %igen Polyacrylamidgel, das carrier ampholyte (2.4%; pH 3-10; Serva) enthält, getrennt. Die Gele wurden aktivitätsgefärbt, mit Trichloroessigsäure-Lösung fixiert (10% (w/v) für 10 min, dann 1 % (w/v) über Nacht) und anschliesslich Coomassie Brilliant Blue gefärbt.
Der isoelektrische Punkt der rekombinanten PLE beträgt pl: 4.78

### Beispiel 10: Esterase-katalysierte Racematspaltung von Acetaten

Zur Esterase-katalysierten Hydrolyse wurden 10 mMol eines Acetates in Natriumphosphatpuffer (pH 7,5, 50 mMol) gelöst und in 1 ml Reaktionsgefäße gegeben und die Racematspaltung durch Zugabe von 0,5 Units (bezogen auf den pNPA-Test) Esterase gestartet. Zum Abbruch der Reaktion wurde das Gemisch mit Methylenchlorid extrahiert und die organische Phase über wasserfreiem Natriumsulfat getrocknet. Die Bestimmung der Enantiomerenreinheit und des Umsatzes erfolgte gaschromatographisch (Säule: Heptakis (2,6-*O*-methyl-3-*O-*pentyl)-β-cyclodextrin, Trägergas: H₂, Flammenionisationsdetektor).

Es wurden die folgenden Acetatsubstrate enzymatisch umgesetzt:

1-Phenyl-1-ethylacetat **1**, 1-Phenyl-2-propylacetat **2**, 1-Phenyl-2-butylacetat **3**, 1-Phenyl-1-propylacetat **4**, 1-Phenyl-3-propylacetat **5**, 1-Phenyl-2-pentylacetat **6**

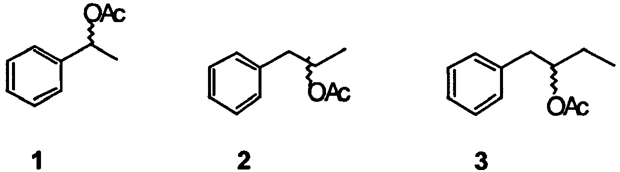

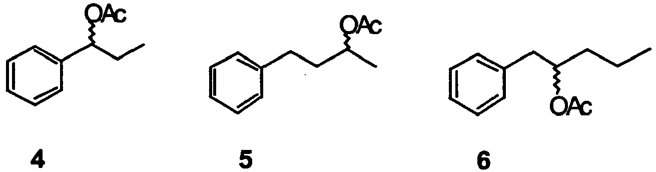

Die Produkte (Alkohole) der kinetischen Racematspaltung mit Schweineleberesterasen werden entsprechend mit **1a-6a** bezeichnet.

Retentionszeiten: **1** (100°C isotherm) : (*S*)-1 3,7 min; (*R*)-1, 5,8 min; (*R*)-**1a** 6,7 min; (*S*)-**1a**, 7,6 min; **2** (75°C isotherm): (*S*)-**2** 26,5 min; (*R*)-**2**, 42,3 min; (*S*)-**2a** 32,6 min;(*R*)-**2a**, 34,2 min. **3** (90°C isotherm): (*S*)-**3** 17,6 min; (*R*)-**3**, 20,2 min; (*S*)-**3a** 24,8 min; (*R*)-**3a**, 27,4 min. **4** (80°C isotherm): (*S*)-**4** 13.9 min; (*R*)-**4** 20.9 min; (*S*)-**4a** 51.5 min; (*R*)-**4a** 44.6 min. **5** (100°C isotherm): (*S*)-**5** 15.6 min; (*R*)-**5** 28.7 min; (*S*)-**5a** 19.1 min; (*R*)-**5a**, 20.5 min. **6** (90°C/ 30'//5°C/min//110°C): (S)-**6**: 29.1 min und (*R*)-**6** 30.4 min; (*S*)-**6a** 37.3 min und (*R*)-**6a** 38.3 min.

Bestimmung der absoluten Konfiguration:
Die absolute Konfiguration basierte für **1** auf einem Vergleich mit käuflichem (*R*)-**1**. Bei **2, 3** und **4** diente die literaturbekannte (*R*)-Präferenz der Lipase Amano PS als Bezug (Gutman, A.L., et. al., Tetrahedron: Assymmetry (1993) 4, 839-844).
Die absolute Konfiguration basierte für **5** auf der literaturbekannten Präferenz der kommerziellen Lipase: Lipase Amano AK, R - preferenz (K. Burgess, LD Jenning, J. Am. Chem. Soc 1991, 113, 6129) für 1-phenyl-3-butanol **(5a)**
Die absolute Konfiguration für **6** basiert auf der Messung des Drehwertes des optisch reinen Alkohols und Vergleich mit den Literaturdaten (U. P. Dhokte, P. M. Pathare, V. K. Mahindroo, H. C. Brown, J. Org. Chem. 1998, 63, 8276-8283).

Die Enantioselektivität E kann gemäß den Chen, C. S. et al., J. Am. Chem. Soc. 104 (1982), 7294 berechnet werden.

Die Ergebnisse sind in den Tabellen 3 bis 8 zusammengefasst.

**Tabelle 3: Enantioselektivität verschiedener Schweineleberesterasen in der kinetischen Racematspaltung von (R,S)-1-Phenyl-1-ethylacetat 1**

| PLE-Quelle^{(a)} | Zeit | Enantiomerenüberschuß | | Umsatz | E^{(b)} |
|---|---|---|---|---|---|
| | (Std.) | (%ee_{S}) | (%ee_{R}) | (%) | |
| Rekombinant | 1 | 58 (*S*) | 53 (*R*) | 53 | 5.7 |
| Fluka | 1,5 | 65 (*S*) | 56 (*R*) | 54 | 6.8 |
| Sigma | 1 | 72 (*S*) | 58 (*R*) | 55 | 7.8 |
| Chirazyme E-1 | 5 | 73 (*S*) | 58 (*R*) | 56 | 7.9 |
| Chirazyme E-2 | 1 | 61 (*S*) | 56 (*R*) | 52 | 6.5 |

| | | | | | |
|---|---|---|---|---|---|
| ^{(a)}in allen Reaktionen wurden 0,5 Units (bezogen auf den pNPA-Test) eingesetzt. ^{(b)}die Enantioselektivität E wurde gemäß Chen et al. (1982) berechnet. | | | | | |

**Tabelle 4: Enantioselektivität verschiedener Schweineleberesterasen in der kinetischen Racematspaltung von (R,S)-1-Phenyl-2-propylacetat 2**

| PLE-Quelle^{(a)} | Zeit | Enantiomerenüberschuß | | Umsatz | E^{(b)} |
|---|---|---|---|---|---|
| | (Std.) | (%ee_{S}) | (%ee_{R}) | (%) | |
| Rekombinant | 2 | 75 (*R*) | 70 (*S*) | 52 | 12.6 |
| Fluka | 1,5 | 35 (*S*) | 44 (*R*) | 44 | 3.6 |
| Sigma | 1,5 | 24 (*S*) | 32 (*R*) | 43 | 2,4 |
| Chirazyme E-1 | 1,5 | 22 (*S*) | 43 (*R*) | 34 | 3.1 |
| Chirazyme E-2 | 1 | 9 (*S*) | 9 (*R*) | 50 | 1.3 |

| | | | | | |
|---|---|---|---|---|---|
| ^{[a], [b]}S. Tabelle 3 | | | | | |

**Tabelle 5: Enantioselektivität verschiedener Schweineleberesterasen in der kinetischen Racematspaltung von (R,S)-1-Phenyl-2-butylacetat 3**

| PLE-Quelle^{(a)} | Zeit | Enantiomerenüberschuß | | | | Umsatz | E^{(b)} |
|---|---|---|---|---|---|---|---|
| | (Std.) | (%ee_{S}) | | (%ee_{R}) | | (%) | |
| Rekombinant | 2 | 57 | (*R*) | >99 | (*S*) | 36 | »100 |
| Fluka | 2 | 12 | (*R*) | 12 | (*S*) | 49 | 1,4 |
| Sigma | 1 | 17 | (*R*) | 11 | (*S*) | 59 | 1,5 |
| Chirazyme E-1 | 2 | 19 | (*R*) | 18 | (*S*) | 52 | 1,7 |
| Chirazyme E-2 | 1 | 58 | (*R*) | 40 | (*S*) | 59 | 4 |

| | | | | | | | |
|---|---|---|---|---|---|---|---|
| ^{[a], [b]}S. Tabelle 3 | | | | | | | |

**Tabelle 6: Enantioselektivität verschiedener Schweineleberesterasen in der kinetischen Racematspaltung von (R,S)-1-Phenyl-1-propylacetat 4**

| PLE^{[a]} | Zeit | Enantiomerenüberschuß | | Umsatz | E^{[b]} |
|---|---|---|---|---|---|
| | [Std.] | [%ee_{S}]^{[c]} | [%ee_{P}]^{[c]} | [%] | |
| Rekombinant | 4 | 13 | 20 | 40 | 1.7 |
| Fluka | 1 | 21 | 28 | 43 | 2.2 |
| Sigma | 0.5 | 17 | 19 | 48 | 1.7 |
| Chirazyme E-1 | 0.5 | 9 | 13 | 41 | 1.4 |
| Chirazyme E-2 | 0.5 | 18 | 27 | 40 | 2.1 |

| | | | | | |
|---|---|---|---|---|---|
| ^{[a], [b]}S. Tabelle 3 ^{[c]}in allen Fällen hatte der Produktalkohol **4a** (R)-Konfiguration und das nichtumgesetzte Acetat **4** (S)-Konfiguration. | | | | | |

**Tabelle 7: Enantioselektivität verschiedener Schweineleberesterasen in der kinetischen Racematspaltung von (R,S)-4-Phenyl-2-butylacetat 5**

| **PLE^{[a]}** | **Zeit** | **Enantiomerenüberschuß** | | **Umsatz** | **E**^{[b]} |
|---|---|---|---|---|---|
| | [Std.] | [%*ee*_{S}] | [%*ee*_{P}] | [%] | |
| Rekombinant | 2 | 52 (R) | 59 (S) | 47 | 6.3 |
| Fluka | 0.5 | 31 (S) | 42 (R) | 42 | 3.2 |
| Sigma | 0.25 | 22 (S) | 25 (R) | 47 | 2.1 |
| Chirazyme E-1 | 0.5 | 25 (S) | 29 (R) | 47 | 2.3 |
| Chirazyme E-2 | 0.25 | 1 (R) | 2 (S) | 43 | 1.1 |

| | | | | | |
|---|---|---|---|---|---|
| ^{[a], [b]}S. Tabelle 3 | | | | | |

**Tabelle 8: Enantioselektivität verschiedener Schweineleberesterasen in der kinetischen Racematspaltung von (R,S)-1-phenyl-2-pentylacetat 6.**

| **PLE^{[a]}** | **Zeit** | **Enantiomerenüberschuß** | | **Umsatz** | **E**^{[b]} |
|---|---|---|---|---|---|
| | [Std.] | [%*ee*_{S}]^{[c]} | [%*ee*_{P}]^{[c]} | [%] | |
| Rekombinant | 2 | 69 | 78 | 47 | 16.7 |
| Fluka | 0.3 | 24 | 26 | 48 | 2.1 |
| Sigma | 0.5 | 15 | 13 | 52 | 1.5 |
| Chirazyme E-1 | 0.5 | 9 | 11 | 46 | 1.3 |
| Chirazyme E-2 | 0.3 | 21 | 24 | 46 | 2.0 |

| | | | | | |
|---|---|---|---|---|---|
| ^{[a], [b]}S. Tabelle 1. ^{[c]}in allen Fällen hatte der Produktalkohol **6a** (S)-Konfiguration und das nichtumgesetzte Acetat **6** (*R*)-Konfiguration. | | | | | |

Das Beispiel zeigt, dass die eingesetzte rekombinante Schweineleberesterase gegenüber Enzymextrakten höhere Enantioselektivität zeigt. Dies wird insbesondere bei Phenyl-Alkyl-Acetaten deutlich, die in 2- oder 3-Stellung verestert sind.

### Beispiel 11: Herstellung der Enzym-Substrate

Die Substrate können nach chemischen Standardverfahren synthetisiert werden. Im folgenden ist die Synthese Substrate (**2**) - (**6**) und der Vorstufe (**6a**) beschrieben. Alle anderen Substrate sind gewerblich erhältlich.

40 mmol Essigsäurechlorid werden in 20 ml Pyridin bei 4°C gelöst. Anschließend werden unter starkem Rühren 40 mMol des Alkohols (1-Phenyl-2-propanol **2a**, 1-Phenyl-2-butanol **3a**, 1-Phenyl-1-propanol **4a**, 1-Phenyl-3-propanol **5a**, 1-Phenyl-2-pentanol **6a**) zugetropft und für 20 Std. bei Raumtemperatur durchgemischt. Danach wird das Reaktionsgemisch mit Diethylether versetzt und zweimal mit gesättigter Natriumhydrogencarbonatlösung gewaschen. Die organische Phase wird über wasserfreiem Natriumsulfat getrocknet und das Lösungsmittel am Rotationsverdampfer entfernt. Das Acetat wird anschließend durch Kieselgelchromatographie (Hexan:Essigester, 5:1) in einer Ausbeute von 91 % (**2**), 74 % (**3**), 43% (**4**) bzw. 87% (**5**) isoliert. Das Acetat (**6**) wurden durch Säulenchromatographie mit Hexan:Essigester 3:1 in einer Ausbeute von 25% (**6**) isoliert.

Synthese von (R,S)-1-phenyl-2-pentanol (**6a**)
In die Grignard-Reagens-Lösung aus 80 mmol Propylmagnesiumchlorid (16 ml etherische Lösung) wird unter Rühren 100 mmol Phenylacetaldehyd im gleichen Volumen abs. Ether zugetropft. Nach beendeter Zugabe wird das Reaktionsgemisch 2 Stunden unter Rühren auf dem Wasserbad erhitzt und danach abgekühlt. Es wird zerstoßenes Eis hinzugegeben und anschließend so viel halbkonz. Salzsäure zugesetzt, dass sich der entstandene Niederschlag löst. Die etherische Schicht wird abgetrennt und die wäßrige Phase noch zweimal mit Ether extrahiert. Die vereinigten Extrakte werden mit gesättigter Natriumhydrogensulfitlösung, Hydrogencarbonatlösung und wenig Wasser gewaschen. Nach dem Trocknen über wasserfreiem Natriumsulfat wird der Ether abdestilliert und der Rückstand durch Destillation fraktioniert. Das Produkt wurde in einer Ausbeute von 48% isoliert.

## Patentansprüche

1. Rekombinante Untereinheit von Schweineleberesterasen, **dadurch gekennzeichnet, dass** die rekombinante Untereinheit eine verkürzte Form eines Proteins mit der Sequenz MWLLPLVLTS LASSATWAGQ PASPPWDTA QGRVLGKYVS LEGLAQPVAV FLGVPFAKPP LGSLRFAPPQ PAEPWSFVKN TTSYPPMCCQ DPVVEQMTSD LFTNGKERLT LEFSEDCLYL NIYTPADLTK RGRLPVMVWI HGGGLVLGGA PMYDGWLAA HENVVVVAIQ YRLGIWGFFS TGDEHSRGNW GHLDQVAALH WVQENIANFG GDPGSVTIFG ESAGGESVSV LVLSPLAKNL FHRAISESGV ALTVALVRKD MKAAAKQIAV LAGCKTTTSA VFVHCLRQKS EDELLDLTLK MKFLTLDFHG DQRESHPFLP TVVDGVLLPK MPEEILAEKD FNTVPYIVGI NKQEFGWLLP TMMGFPLSEG KLDQKTATSL LWKSYPIANI PEELTPVATD KYLGGTDDPV KKKDLFLDLM GDWFGVPSV TVARQHRDAG APTYMYEFQY RPSFSSDKKP KTVIGDHGDE IFSVFGFPLL KGDAPEEEVS LSKTVMKFWA NFARSGNPNG EGLPHWPMYD QEEGYLQIGV NTQAAKRLKG EEVAFWNDLL SKEAAKKPPK IKHAEL darstellt, das am C-terminalen Ende um 3 bis 10 Aminosäuren verkürzt ist, oder eine funktionelle mutante Form des Proteins gemäß SEQ ID NO: 1 mit einer Sequenzhomologie von über 80% dazudarstellt.

2. Rekombinante Untereinheit von Schweineleberesterasen nach Anspruch 1, wobei die rekombinante Untereinheit ein Protein gemäß SEQ ID NO: 1 darstellt.

3. Rekombinante Untereinheit einer Schweineleberesterase gemäß Anspruch 1 oder 2 **dadurch gekennzeichnet, dass** der Untereinheit am N-terminalen Ende 15 bis 25 Aminosäuren fehlen.

4. Rekombinante Untereinheit von Schweineleberesterasen gemäß einem der vorherigen Ansprüche, **dadurch gekennzeichnet, dass** N-terminal eine Sekretionssignal-Domäne und/oder C-terminal eine Tag-Domäne fusioniert ist.

5. Rekombinante Untereinheit von Schweineleberesterasen gemäß Anspruch 4, **dadurch gekennzeichnet, dass** als N-terminale Domäne eine α-Faktor-Signalsequenz, deren kodierende Sequenz in SEQ ID NO: 8 enthalten ist, oder eine ompA-Signalsequenz, deren kodierende Sequenz in SEQ ID NO: 10 enthalten ist, und/oder C-terminal eine poly-His-Tag- und/oder eine myc-Tag-Domäne fusioniert ist.

6. Rekombinante Untereinheit von Schweineleberesterasen gemäß einem der vorhergehenden Ansprüche, **dadurch gekennzeichnet, dass** die Untereinheiten posttranslationale Modifikationen aufweisen.

7. DNA codierend für eine Schweineleberesterase-Untereinheit gemäß einem der vorherigen Ansprüche.

8. DNA codierend für eine Schweineleberesterase-Untereinheit gemäß Anspruch 6, **dadurch gekennzeichnet, dass** sie am 3'-Ende des codierenden Bereichs ein Stop-Codon enthält.

9. Vektoren enthaltend DNA-Fragment mit einer Nukleinsäuresequenz gemäß Anspruch 7 oder 8.

10. Enzymatisch aktive rekombinante Schweineleberesterasen enthaltend Schweineleberesterasen-Untereinheiten gemäß einem der Ansprüche 1 bis 6.

11. Enzymatisch aktive rekombinante Schweineleberesterasen gemäß Anspruch 10, **dadurch gekennzeichnet, dass** die Untereinheiten posttranslationale Modifikationen aufweisen.

12. Verwendung von DNA-Fragmenten gemäß Anspruch 7 oder 8 zur Herstellung von Expressionsvektoren.

13. Verwendung von Vektoren gemäß Anspruch 9 zur Herstellung von transfizierten Zellkulturen.

14. Verwendung von DNA-Fragmenten gemäß den Ansprüchen 7 oder 8 oder Vektoren gemäß dem Anspruch 9 zur Herstellung von mutanten rekombinanten Schweineleberesterase-Untereinheiten und funktionsfähigen Enzymen.

15. Expression von Schweineleberesterase-Untereinheiten gemäß den Ansprüchen von 1 bis 6 mit Zellkulturen enthaltend Vektoren gemäß Anspruch 9.

16. Expression von enzymatisch aktiven Schweineleberesterasen gemäß einem der Ansprüche 10 oder 11 mit Zellkulturen enthaltend Vektoren gemäß Anspruch 9.

17. Expression von enzymatisch aktiven Schweineleberesterasen gemäß einem der Ansprüche 15 oder 16 **dadurch gekennzeichnet, dass** die genutzten Zellkulturen einzellige pro- oder eukaryotische Organismen sind.

18. Verwendung von Schweineleberesterasen gemäß einem der Ansprüche 10 oder 11 zur Racematspaltung von Carbonsäuren oder bei der Umsetzung von prostereogenen Verbindungen.

## Claims

1. A recombinant subunit of porcine liver esterases, **characterized in that** the recombinant subunit is a truncated form of a protein having the sequence which is truncated by 3 to 10 amino acids at the C terminus, or a functional mutant form of the protein of SEQ ID NO:1 having a sequence homology of more than 80% thereto.

2. The recombinant subunit of porcine liver esterases according to claim 1, wherein the recombinant subunit is a protein of SEQ ID NO:1.

3. The recombinant subunit of a porcine liver esterase according to claim 1 or 2, **characterized in that** the subunit lacks15 to 25 amino acids at the N terminus.

4. The recombinant subunit of porcine liver esterases according to any one of the preceding claims, **characterized in that** a secretion signal domain is fused at the N terminus and/or a tag domain is fused at the C terminus.

5. The recombinant subunit of porcine liver esterases according to claim 4, **characterized in that** an α factor signal sequence the coding sequence of which is contained in SEQ ID NO:8 or an ompA signal sequence the coding sequence of which is contained in SEQ ID NO:10 is fused as an N-terminal domain, and/or a poly-His-tag domain and/or a myc-tag domain is/are fused C-terminally.

6. The recombinant subunit of porcine liver esterases according to any one of the preceding claims, **characterized in that** the subunits have post-translational modifications.

7. A DNA encoding a porcine liver esterase subunit according to any one of the preceding claims.

8. A DNA encoding a porcine liver esterase subunit according to claim 6, **characterized in that** it contains a stop codon at the 3' end of the coding region.

9. Vectors containing a DNA fragment with a nucleic acid sequence according to claim 7 or 8.

10. Enzymatically active recombinant porcine liver esterases containing porcine liver esterase subunits according to any one of claims 1 to 6.

11. The enzymatically active recombinant porcine liver esterases according to claim 10, **characterized in that** the subunits have post-translational modifications.

12. Use of DNA fragments according to claim 7 or 8 for the preparation of expression vectors.

13. Use of vectors according to claim 9 for the preparation of transfected cell cultures.

14. Use of DNA fragments according to claims 7 or 8 or vectors according to claim 9 for the preparation of mutant recombinant porcine liver esterase subunits and functional enzymes.

15. Expression of porcine liver esterase subunits according to claims 1 to 6 using cell cultures containing vectors according to claim 9.

16. The expression of enzymatically active porcine liver esterases according to any one of claims 10 or 11 using cell cultures containing vectors according to claim 9.

17. The expression of enzymatically active porcine liver esterases according to any one of claims 15 or 16, **characterized in that** the cell cultures used are uni-cellular prokaryotic or eukaryotic organisms.

18. Use of porcine liver esterases according to any one of claims 10 or 11 for racemate resolution of carboxylic acids or in the conversion of prostereogenic compounds.

## Revendications

1. Sous-unité recombinante d'estérases de foie de porc, **caractérisée en ce que** la sous-unité recombinante représente une forme raccourcie d'une protéine ayant la qui est raccourcie de 3 à 10 acides aminés au niveau de l'extrémité C-terminale, ou qui représente une forme mutante fonctionnelle de la protéine selon SEQ ID N°1 avec une homologie de séquence de plus de 80 %.

2. Sous-unité recombinante d'estérases de foie de porc selon la revendication 1, dans laquelle la sous-unité recombinante représente une protéine selon SEQ ID N°1.

3. Sous-unité recombinante d'une estérase de foie de porc selon la revendication 1 ou 2, **caractérisée en ce que** la sous-unité est dépourvue de 15 à 25 acides aminés au niveau de l'extrémité N-terminale.

4. Sous-unité recombinante d'estérases de foie de porc selon l'une des revendications précédentes, **caractérisée en ce qu'**un domaine signal de sécrétion est fusionné au niveau de l'extrémité N-terminale et/ou un domaine Tag est fusionné au niveau de l'extrémité C-terminale.

5. Sous-unité recombinante d'estérases de foie de porc selon la revendication 4, **caractérisée en ce qu'**une séquence signal du facteur α, dont la séquence codante est comprise dans SEQ ID N°8, ou une séquence signal ompA, dont la séquence codante est comprise dans SEQ ID N°10, est fusionnée comme domaine N-terminal, et/ou un domaine poly-His-Tag et/ou myc-Tag est/sont fusionné(s) au niveau de l'extrémité C-terminale.

6. Sous-unité recombinante d'estérases de foie de porc selon l'une des revendications précédentes, **caractérisée en ce que** les sous-unités présentent des modifications post-translationnelles.

7. ADN codant pour une sous-unité d'estérases de foie de porc selon l'une des revendications précédentes.

8. ADN codant pour une sous-unité d'estérases de foie de porc selon la revendication 6, **caractérisé en ce qu'**il contient un codon stop au niveau de l'extrémité 3' du domaine codant.

9. Vecteurs comprenant un fragment d'ADN ayant une séquence d'acide nucléique selon la revendication 7 ou 8.

10. Estérases de foie de porc recombinantes enzymatiquement actives comprenant des sous-unités d'estérases de foie de porc selon l'une des revendications 1 à 6.

11. Estérases de foie de porc recombinantes enzymatiquement actives selon la revendication 10, **caractérisées en ce que** les sous-unités présentent des modifications post-translationnelles.

12. Utilisation de fragments d'ADN selon la revendication 7 ou 8 pour la préparation de vecteurs d'expression.

13. Utilisation de vecteurs selon la revendication 9 pour la préparation de cultures cellulaires transfectées.

14. Utilisation de fragments d'ADN selon les revendications 7 ou 8 ou de vecteurs selon la revendication 9 pour la préparation de sous-unités d'estérases de foie de porc recombinantes mutantes et d'enzymes fonctionnelles.

15. Expression de sous-unités d'estérases de foie de porc selon les revendications 1 à 6 avec des cultures cellulaires contenant des vecteurs selon la revendication 9.

16. Expression d'estérases de foie de porc enzymatiquement actives selon l'une des revendications 10 ou 11 avec des cultures cellulaires contenant des vecteurs selon la revendication 9.

17. Expression d'estérases de foie de porc enzymatiquement actives selon l'une des revendications 15 ou 16, **caractérisée en ce que** les cultures cellulaires utilisées sont des organismes procaryotes ou eucaryotes unicellulaires.

18. Utilisation d'estérases de foie de porc selon l'une des revendications 10 ou 11 pour le clivage de la forme racémique des acides carboxyliques ou lors de la mise en oeuvre de composés prostéréogéniques.


### SEQUENZPROTOKOLL

<110> Degussa AG
<120> Rekombinante Schweineleberesterasen, deren Verwendung sowie ein Verfahren zu deren Herstellung
<130> 200at29.wo
<140>
   <141>
<150> DE 100 61 864.2
   <151> 2000-12-12
<160> 12
<170> PatentIn Ver. 2.1
<210> 1
   <211> 544
   <212> PRT
   <213> Künstliche Sequenz
<220>
   <223> Beschreibung der künstlichen Sequenz: rekombinante Enzymuntereinheit
<400> 1
   Gly Gln Pro Ala Ser Pro Pro Val Val Asp Thr Ala Gln Gly Arg Val
    1               5                  10                  15
   Leu Gly Lys Tyr Val Ser Leu Glu Gly Leu Ala Gln Pro Val Ala Val
               20                  25                  30
   Phe Leu Gly Val Pro Phe Ala Lys Pro Pro Leu Gly Ser Leu Arg Phe
           35                  40                  45
   Ala Pro Pro Gln Pro Ala Glu Pro Trp Ser Phe Val Lys Asn Thr Thr
       50                  55                  60
   Ser Tyr Pro Pro Met Cys Cys Gln Asp Pro Val Val Glu Gln Met Thr
   65                  70                  75                  80
   Ser Asp Leu Phe Thr Asn Gly Lys Glu Arg Leu Thr Leu Glu Phe Ser
                   85                  90                  95
   Glu Asp Cys Leu Tyr Leu Asn Ile Tyr Thr Pro Ala Asp Leu Thr Arg
   100 105 110
   Arg Gly Arg Leu Pro Val Met Val Trp Ile His Gly Gly Gly Leu Val
   115 120 125
   Leu Gly Gly Ala Pro Met Tyr Asp Gly Val Val Leu Ala Ala His Glu
   130 135 140
   Asn Val Val Val Val Ala Ile Gln Tyr Arg Leu Gly Ile Trp Gly Phe
   145 150 155 160
   Phe Ser Thr Gly Asp Glu His Ser Arg Gly Asn Trp Gly His Leu Asp
   165 170 175
   Gln Val Ala Ala Leu His Trp Val Gln Glu Asn Ile Ala Asn Phe Gly
   180 185 190
   Gly Asp Pro Gly Ser Val Thr Ile Phe Gly Glu Ser Ala Gly Gly Glu
   195 200 205
   Ser Val Ser Val Leu Val Leu Ser Pro Leu Ala Lys Asn Leu Phe His
   210 215 220
   Arg Ala Ile Ser Glu Ser Gly Val Ala Leu Thr Val Ala Leu Val Arg
   225 230 235 240
   Lys Asp Met Lys Ala Ala Ala Lys Gln Ile Ala Val Leu Ala Gly Cys
   245 250 255
   Lys Thr Thr Thr Ser Ala Val Phe Val His Cys Leu Arg Gln Lys Ser
   260 265 270
   Glu Asp Glu Leu Leu Asp Leu Thr Leu Lys Met Lys Phe Leu Thr Leu
   275 280 285
   Asp Phe His Gly Asp Gln Arg Glu Ser His Pro Phe Leu Pro Thr Val
   290 295 300
   Val Asp Gly Val Leu Leu Pro Lys Met Pro Glu Glu Ile Leu Ala Glu
   305 310 315 320
   Lys Asp Phe Asn Thr Val Pro Tyr Ile Val Gly Ile Asn Lys Gln Glu
   325 330 335
   Phe Gly Trp Leu Leu Pro Thr Met Met Gly Phe Pro Leu Ser Glu Gly
   340 345 350
   Lys Leu Asp Gln Lys Thr Ala Thr Ser Leu Leu Trp Lys Ser Tyr Pro
   355 360 365
   Ile Ala Asn Ile Pro Glu Glu Leu Thr Pro Val Ala Thr Asp Lys Tyr
   370 375 380
   Leu Gly Gly Thr Asp Asp Pro Val Lys Lys Lys Asp Leu Phe Leu Asp
   385 390 395 400
   Leu Met Gly Asp Val Val Phe Gly Val Pro Ser Val Thr Val Ala Arg
   405 410 415
   Gln His Arg Asp Ala Gly Ala Pro Thr Tyr Met Tyr Glu Phe Gln Tyr
   420 425 430
   Arg Pro Ser Phe Ser Ser Asp Lys Lys Pro Lys Thr Val Ile Gly Asp
   435 440 445
   His Gly Asp Glu Ile Phe Ser Val Phe Gly Phe Pro Leu Leu Lys Gly
   450 455 460
   Asp Ala Pro Glu Glu Glu Val Ser Leu Ser Lys Thr Val Met Lys Phe
   465 470 475 480
   Trp Ala Asn Phe Ala Arg Ser Gly Asn Pro Asn Gly Glu Gly Leu Pro
   485 490 495
   His Trp Pro Met Tyr Asp Gln Glu Glu Gly Tyr Leu Gln Ile Gly Val
   500 505 510
   Asn Thr Gln Ala Ala Lys Arg Leu Lys Gly Glu Glu Val Ala Phe Trp
   515 520 525
   Asn Asp Leu Leu Ser Lys Glu Ala Ala Lys Lys Pro Pro Lys Ile Lys
   530 535 540
<210> 2
   <211> 34
   <212> DNA
   <213> Künstliche Sequenz
<220>
   <223> Beschreibung der künstlichen Sequenz: Primer
<400> 2
   gatatcccgg gcatatgtgg cttctcccgc tggt 34
<210> 3
   <211> 35
   <212> DNA
   <213> Künstliche Sequenz
<220>
   <223> Beschreibung der künstlichen Sequenz: Primer
<400> 3
   gcatcccggg aattctcaca gctcagcatg cttta 35
<210> 4
   <211> 31
   <212> DNA
   <213> Künstliche Sequenz
<220>
   <223> Beschreibung der künstlichen Sequenz: Primer
<400> 4
   gggcagccag cctcgccgcc tgttgtggac a 31
<210> 5
   <211> 31
   <212> DNA
   <213> Künstliche Sequenz
<220>
   <223> Beschreibung der künstlichen Sequenz: Primer
<400> 5
   tcacagctca gcatgcttta tcttgggtgg c 31
<210> 6
   <211> 23
   <212> DNA
   <213> Künstliche Sequenz
<220>
   <223> Beschreibung der künstlichen Sequenz: Primer
<400> 6
   agcctgcgct acggtagcga aac 23
<210> 7
   <211> 22
   <212> DNA
   <213> Künstliche Sequenz
<220>
   <223> Beschreibung der künstlichen Sequenz: Primer
<400> 7
   tgaagggatc ctaagtaagt ag 22
<210> 8
   <211> 32
   <212> DNA
   <213> Künstliche Sequenz
<220>
   <223> Beschreibung der künstlichen Sequenz: Primer
<400> 8
   aagctgaatt cgggcagcca gcctcgccgc ct 32
<210> 9
   <211> 33
   <212> DNA
   <213> Künstliche Sequenz
<220>
   <223> Beschreibung der künstlichen Sequenz: Primer
<400> 9
   gtcagtctag atcacagctc agcatgcttt atc 33
<210> 10
   <211> 33
   <212> DNA
   <213> Künstliche Sequenz
<220>
   <223> Beschreibung der künstlichen Sequenz: Primer
<400> 10
   aagctgaatt cgggcagcca gcctcgccgc ctg 33
<210> 11
   <211> 30
   <212> DNA
   <213> Künstliche Sequenz
<220>
   <223> Beschreibung der künstlichen Sequenz: Primer
<400> 11
   acctctagat actttatctt gggtggcttc 30
<210> 12
   <211> 31
   <212> DNA
   <213> Künstliche Sequenz
<220>
   <223> Beschreibung der künstlichen Sequenz: Primer
<400> 12
   acctctagat cactttatct tgggtggctt c 31